# EUROPEAN PATENT SPECIFICATION

(11) **EP 1 781 589 B1**
(45) Date of publication and mention of the grant of the patent: **14.04.2010**
(21) Application number: 05791316.2
(22) Date of filing: 23.08.2005
(51) Int. Cl.: C07C 46/00, C07C 50/38

(54) **SYNTHESIS OF IDARUBIN AGLYCONE**
SYNTHESE VON IDARUBIN-AGLYCON
SYNTHESE D'AGLYCONE D'IDARUBICINE

(30) Priority: 23.08.2004 US 604038 P; 01.09.2004 US 606813 P
(43) Date of publication of application: 09.05.2007
(73) Proprietor: Sicor Inc., Irvine, CA 92618 (US)
(72) Inventor: VILLA, Marco, 20125 Milano (IT); AROSIO, Roberto, Civate (LC) (IT); FRETTA, Roberta, Collegno, Torino (IT); DIULGHEROFF, Nicola, Torino (IT)
(74) Representative: Gallagher, Kirk James
(86) International application number: PCT/US2005/030298
(87) International publication number: WO 2006/024016

(56) References cited:
- EP-A- 0 337 665
- EP-A- 0 385 354
- WO-A-01/87814
- DATABASE CAPLUS [Online] CHEMICAL ABSTRACTS SERVICE, COLUMBUS, OHIO, US; TSUCHIDA, T. ET AL.: "Preparation of 4-demethoxydaunomycinone for anticancers" XP002366191 retrieved from STN Database accession no. 137:216823 -& JP 2002 255888 A (MERCIAN CORP., JAPAN) 11 September 2002 (2002-09-11)
- CABRI W ET AL: "A NOVEL SYNTHESIS OF (+)-4-DEMETHOXYDAUNOMYCINONE" JOURNAL OF THE CHEMICAL SOCIETY, PERKIN TRANSACTIONS 1, CHEMICAL SOCIETY. LETCHWORTH, GB, vol. 2, no. 1, 1990, pages 428-429, XP009044858 ISSN: 0300-922X

## Description

### FIELD OF THE INVENTION

The present invention relates to an improved method for preparing idarubicin aglycone.

### BACKGROUND OF THE INVENTION

(7S, 9S) 9-acetyl-7,8,9,10-tetrahydro-6,7,9,11-tetrahydroxy-5,12-naphthacenedione, (Idarubicin aglycon or 4-demethoxydaunomycinone) having the formula, 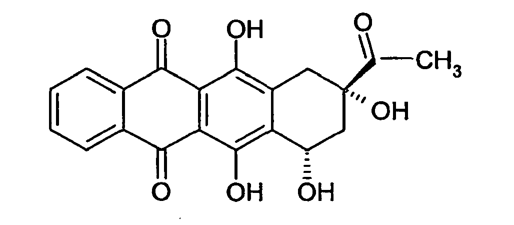 is a derivative of (7S, 9S)-7-[(3-amino-2,3,6-trideoxy-(alpha)-L-lyxo-hexopyranosyl)oxy]-7,8,9,1 0-tetrahydro-6,9,1 1 -trihydroxy -9-acetyl- 5,12-naphthacenedione having the following formula.

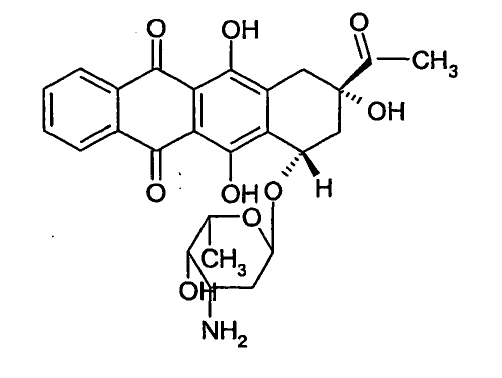

International Patent Publication WO 01/87814 discloses a process for preparing 4-demethyldaunomycinone-4-triflate followed by its reduction to idarubicin aglycone. WO 01/87814 describes reduction of 4-demethyldaunomycinone-4-triflate (0.012 moles) with triethylamine (0.045 moles), 99% formic acid (0.040 moles), 1,1'bis-(diphenylphosphino)ferrocene (0.0005 moles) and Pd(OAc)₂ (0.0005 moles), in dioxane, at 40°C. The yield reported is 66%, but no information about the product purity, or description of possible purification steps, are given. The products of the reaction described in WO 01/87814 would be expected to be very difficult to clean up, likely requiring chromatography steps that are not very convenient to carry out on an industrial scale.

U.S. Patent No. 5,103,029 discloses a process in which 4-demethoxydaunomycinone is obtained with a yield of 71.6% and 98% purity (HPLC), but only after chromatographic purification of the final crude product. The necessity for significant purification steps was not unexpected, since impurities (e.g., 4-demethyldaunomycinone, and products of aromatization of the anthracycline A ring) typically form when the described reductions are conducted on this kind of substrate. Such impurities are usually present in large amounts and/or are not easily removed from the crude product because of their chemical and physical properties, usually requiring chromatographic purifications. Thus, according to these prior art processes, purification steps applied to the crude 4-demethoxydaunomycinone are usually necessary, and strongly reduce the overall yield of the processes.

U.S. Patent No. 5,015,745 discloses a method of making 4-demethoxydaunomycinone from demethyldaunomycinone comprising the steps of protecting the 13-keto group, sulfonylating the 4-OH group, reacting the sulfonylated compound to produce an amine at the 4 position, diazotizing the 4-amine, and reducing under mild conditions to give the final demethoxy compound.

JP 2002 255 888 discloses the preparation of 4-demethoxydaunomycinone by reducing 4-demethyl-4-trifluoromethylsulfonyldaunomycinone with triethylsilane in the presence of a catalyst in a polar aprotic solvent.

Therefore, there is a need for a new process for the reduction of the triflate group to obtain pure idarubicin aglycone, wherein the amount of the undesired byproducts is small.

### SUMMARY OF THE INVENTION

One aspect of the present invention is a process for the preparation of idarubicin aglycone (4-demethoxydaunomycinone) of formula I 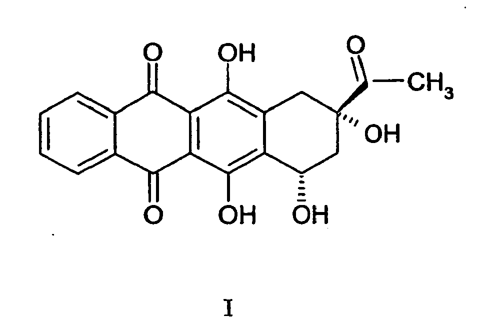 comprising the steps of
(a) combining the corresponding sulfonate of formula II 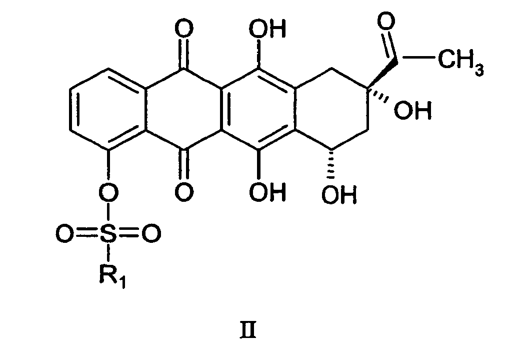 with a first polar aprotic organic solvent and with a metal catalyst or co-catalyst to obtain a first solution;
(b) combining 0.9 to 1.3 mole equivalents of the silyl reagent of the formula R₂R₃R₄SiH with a base and a second polar aprotic organic solvent, with or without a protic solvent to obtain a second solution;
(c) adding the second solution of step (b) to the first solution of step (a) to obtain a mixture, and
(d) adding a solution of the silyl reagent of the formula R₂R₃R₄SiH in a second polar aprotic organic solvent to the mixture of step (c), when no more than 96% area by HPLC of the sulfonate of formula II has reacted, and
(e) maintaining the mixture obtained in step (d) for at least 20 minutes;
(f) quenching the mixture maintained in step (e); and
(g) recovering the idarubicin aglycone of formula I;
wherein R₁ is C₁₋₁₀ alkyl, C₁₋₁₀ alkyl substituted with halogens, an aryl group, an aryl group substituted with halogen or an electron withdrawing group, and R₂, R₃, and R₄ are independently branched or linear C₁₋₄ alkyl, aryl, heteroaryl groups, or polymethylsiloxane.

Preferably, R₁ is C₁₋₁₀ alkyl fully substituted with halogens, and, more preferably, is CF₃.

Preferably, R₂, R₃ and R₄ are the same alkyl groups, and, more preferably, R₂, R₃ and R₄ are ethyl groups.

Preferably, a co-catalyst is used in step (a).

Preferably, the silyl reagent of the formula R₂R₃R₄SiH is used in step (b) in an amount of 1.15 mole equivalent per mole equivalent of the sulfonate of the formula II.

Preferably, the second solution of step (b) is added to the first solution of step (a) in a dropwise manner. More preferably, the dropwise addition is done over a period of 20 minutes to 1.5 hours.

Preferably, the solution of step (b) also contains a protic solvent. Preferably, the protic solvent is either a C₁₋₅ alcohol or water. Preferably, the C₁₋₅ alcohol is methanol. The more preferred protic solvent is water.

Preferably, the amount of the protic solvent is of 0.1 mole equivalents to 5 mole equivalents per mole equivalent of the sulfonate of formula II.

Idarubicin aglycone of formula I may be purified by a process of crystallization from a mixture of a solvent and an anti-solvent. The solvent is preferably, a polar organic solvent, selected from the group consisting of dichloromethane, acetone, acetonitrile and THF. The anti-solvent is preferably a non-polar organic solvent, more preferably, diisopropylether or toluene. Most preferably, the mixture of a solvent and an anti solvent contains acetonitrile with diisopropylether or THF with toluene.

Idarubicin aglycone obtained by the above crystallization process contains less than 1% area by HPLC, of undesired by products.

Preferably, idarubicin aglycone obtained by the above crystallization process contains less than 0.1% area by HPLC of 4-hydroxy derivative of formula IV.

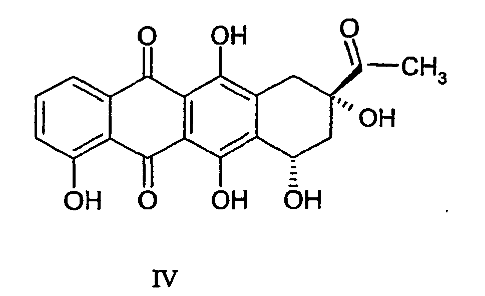

Idarubicin aglycone obtained by the process of this invention may be converted to pharmaceutically acceptable salts of idarubicin, preferably, idarubicin hydrochloride.

### BRIEF DESCRIPTION OF THE DRAWINGS

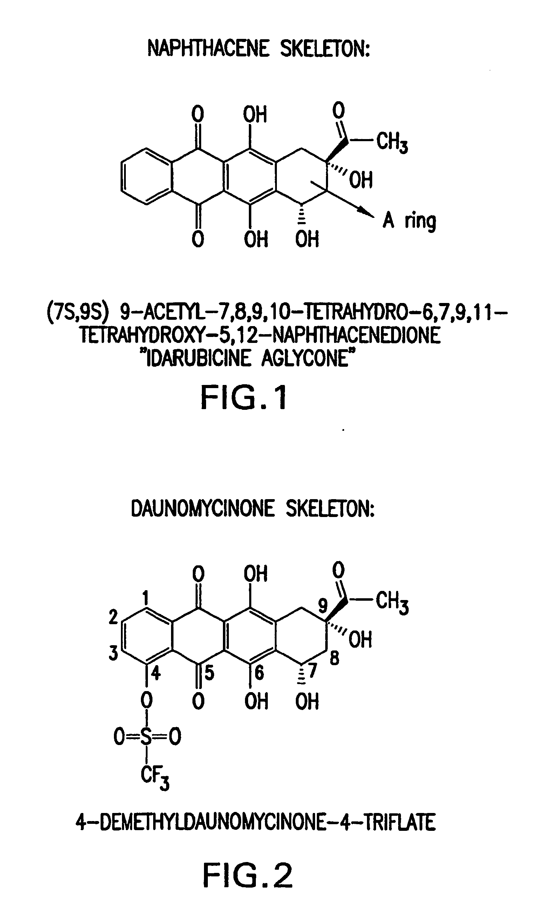

**Figure 1****:** Structure and carbon skeleton numbering of idarubicin aglycone (7S, 9S) 9-acetyl-7,8,9,10-tetrahydro-6,7,9, -tetrahydroxy-5,12-naphthacenedione.
**Figure 2****:** Structure and carbon skeleton numbering of 4-demethyldaunomycinone-4-triflate.

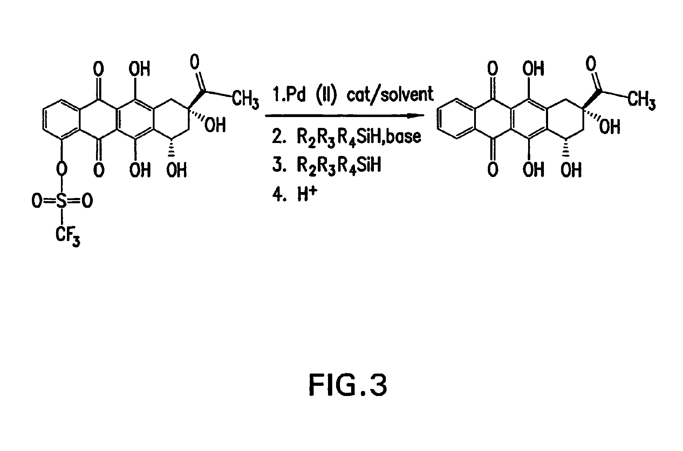

**Figure 3****:** Schematic flow diagram of a reaction of the present invention.

### DETAILED DESCRIPTION OF THE INVENTION

The term "heteroaryl" refers to an aryl group that includes from one to four heteroatoms, for example, pyrrole, furan, thiophene, imidazole, oxazole, thiazole, triazole, pyrazole, pyridine, pyrazine, pyridazine, pyrimidine, and the like. The term "heteroatom," as used herein, means an atom of any element other than carbon or hydrogen.

As used herein, the term electron "withdrawing group" refers to a single atom or to a group of atoms that cause the electron density of the chemical bond to shift toward them, for example, NO₂, COOR, COOH, CO, SO₂, CO, and C₁₋₁₀ alkyl fully substituted with halogens.

As used herein, the term "weak organic base" refers to a negatively charged single atom or a group of atoms that have low affinity to H⁺, for example, pyridine, substituted pyridine, Et₃N, Bu₃N and ethyldiisopropylamine.

As used herein, the term "hindered organic base" refers to a negatively charged single atom or a group of atoms substituted with bulky groups, for example, Et₃N, 2,6-dimethylpyridine, diisopropylethylamine and tributylamine.

As used herein, the term "co-catalyst" refers to the non-active form of the catalyst, which is transformed to the active form by a reaction.

In the process of the present invention the 13-keto group of the 4-demethyldaunomycinone-4-triflate does not have to be protected prior to conversion of the 4-OH group to the 4-demethoxy, as in the prior art processes described in U.S. Patent No. 5,015,745 and in European Patent Application 0 337 665.

In the process of the present invention, idarubicin aglycone (4-demethoxydaunomycinone) is obtained in much higher yields and with greater purity than with prior art methods. In particular, the formation of those impurities that typically occurs when reductive processes are conduced on this kind of substrate, represented by the compound of the formula III, 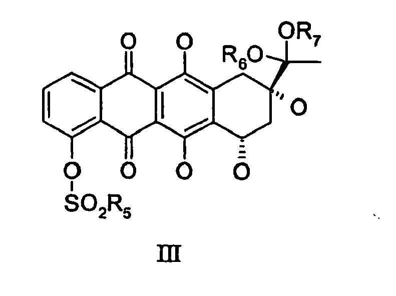 wherein, R₆ and R₇ can be the same, preferably C₁-C₁₀ alkyl or -CH₂-CH₂-, are drastically reduced. Under the conditions of the invention, impurities are present in minimal amounts, so that the crude final product does not need any subsequent onerous purification steps, such as chromatography. Thus, the high quality of the crude idarubicin aglycone allows the use of a very simple crystallization procedure - if needed - which leads to a high quality product in a very high yield.

The present invention provides a process for the preparation of pure idarubicin aglycone (4-demethoxydaunomycinone) of formula I 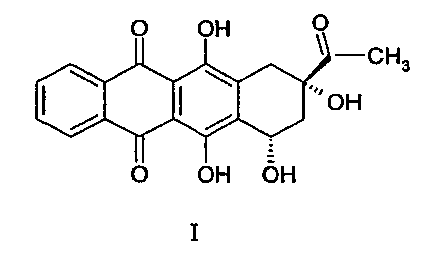 comprising the steps of
(a) combining the corresponding sulfonate of formula II, 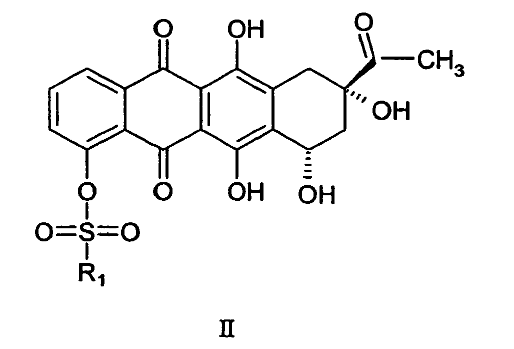 with a first polar aprotic organic solvent and with a metal catalyst or co-catalyst to obtain a first solution;
(b) combining 0.9 to 1.3 mole equivalents of the silyl reagent of the formula R₂R₃R₄SiH with a base, and a second polar aprotic organic solvent, with or without a protic solvent to obtain a second solution;
(c) adding the second solution of step (b) to the first solution of step (a) to obtain a mixture, and
(d) adding a solution of the silyl reagent of the formula R₂R₃R₄SiH in a second polar aprotic organic solvent to the mixture of step (c), when no more than 96% area by HPLC of the sulfonate of formula II has reacted; and
(e) maintaining the mixture obtained in step (d) for at least 20 minutes;
(f) quenching the mixture maintained in step (e), and
(g) recovering the idarubicin aglycone of the formula I;
wherein R₁ is C₁₋₁₀ alkyl, C₁₋₁₀ alkyl substituted with halogen, an aryl group, an aryl group substituted with halogen or an electron withdrawing group; and R₂, R₃, and R₄ are independently branched or linear C₁₋₄ alkyl, aryl, heteroaryl groups or polymethylsiloxane.

Preferably, R₁ is C₁₋₁₀ alkyl fully substituted with halogen, and, more preferably, is CF₃. The compound of formula II corresponds to 4-demethyldaunomycinone-4-triflate having the following formula.

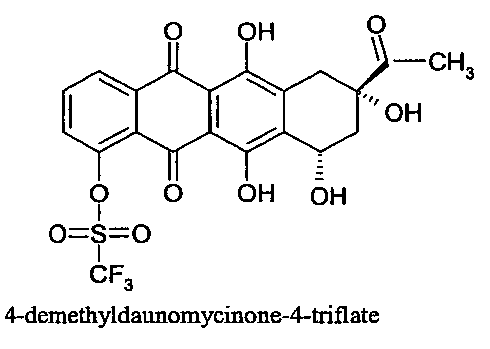

Preferably, R₂, R₃ and R₄ are the same alkyl groups, and, more preferably, R₂, R₃ and R₄ are ethyl groups. Preferably, the compound of the formula R₂R₃R₄SiH is triethylsilane.

Preferably, Et₃SiH is commercially available.

The 4-demethoyldaunomycinone-4-triflate of formula II may be prepared, for example, according to the process disclosed in WO 01/87814.

The temperature of step (a) is preferably from 10°C to 46°C, and, more preferably, is from 15°C to 25°C.

The first polar aprotic organic solvent used in step (a) is selected from the group consisting of amide, ether and ketone. A preferred amide is dimethylformamide (DMF), dimethylacetamide, or N-methylpyrrolidinone. Preferably, the ether is a cyclic ether, more preferably, tetrahydrofuran (THF) or 2-methyl-THF. A preferred ketone is acetone. More preferably, the first polar aprotic organic solvent is DMF. Preferably, a co-catalyst is used in step (a).

The metal co-catalyst is preferably, dichlorobis(triphenylphosphine)Ni(II), dichlorobis(triphenylphosphine)Pd(II), or Pd(II)(OAc)₂ in the presence of triphenylphosphine, and, more preferably, is dichlorobis(triphenylphosphine)Pd(II), which is reduced in situ to a give the active catalyst [bis(triphenylphosphine)] Pd(0).

Preferably, the silyl reagent of formula R₂R₃R₄SiH is used in step (b) in an amount of 1.15 mole equivalent per mole equivalent of the sulfonate of formula II.

The base used in step (b) is preferably, a weak organic base, more preferably, a hindered base, selected from the group consisting of pyridine, 2,6-dimethylpyridine, diisopropylethylamine, triethylamine, tributylamine, and imidazole. Most preferably, the base is 2,6-dimethylpyridine. The use of a weak base, such as 2,6-dimethylpyridine, instead of strong bases limits the amount of byproducts that can be produced in the reaction.

The second polar aprotic organic solvent in step (b) and in step (d) is preferably the same as the aprotic organic solvent in step (a); more preferably, the second polar aprotic organic solvent is DMF.

Preferably, the second solution of step (b) is added to the first solution of step (a) in a dropwise manner. More preferably, the dropwise addition was done over a period of 20 minutes to 1.5 hours.

The dropwise addition of the above solution allows an accurate control of the silane quantity present in the reaction mixture. An excess of silane at prolonged reaction times induces the formation of over-reduced species, while a lack of silane prevents the complete conversion of starting material. Therefore, the reaction progress is monitored by HPLC, and, when no more than 96% area by HPLC, of the sulfonate of formula II has reacted, a second amount of the silane solution is added.

Preferably, the solution of step (b) also contains a protic solvent to avoid the formation of some impurities, such as the 7-deoxy derivative of formula V. Preferably, the protic solvent is either C₁₋₅ alcohol or water. Preferably, the C₁₋₅ alcohol is methanol. The more preferred protic solvent is water.

Preferably, the amount of the protic solvent is 0.1 mole equivalents to 5 mole equivalents per mole equivalent of the sulfonate of formula II.

The solution of the silane of formula R₂R₃R₄S1H in the second polar aprotic organic solvent is added in step (d) to bring the reaction to completion.

The mixture obtained in step (d) is maintained preferably, for 20 minutes to 2 hours, depending on the reaction temperature.

Preferably, quenching may be done using an acidic aqueous solution, more preferably, HCl, acetic acid, or ammonium chloride. Most preferably, quenching may be done using aqueous HEl.

Idarubicin aglycone of formula I is recovered by any method known in the art, such as precipitating by the addition of water, filtering and washing the obtained solid with a polar organic solvent or with water.

Idarubicin aglycone of formula I may be further purified by a process of crystallization from a mixture of a solvent and an anti-solvent. The solvent is preferably, a polar organic solvent, selected from the group consisting of dichloromethane, acetone, acetonitrile and THF. The anti-solvent is preferably a non-polar organic solvent; more preferably, diisopropylether or toluene. Most preferably, the mixture of a solvent and an anti-solvent contains acetonitrile with diisopropylether or THF with toluene.

Idarubicin aglycone obtained by the above crystallization process contains less than about 1% area by HPLC, of undesired byproducts. Such byproducts may be one of: 4-hydroxy derivative of formula IV, 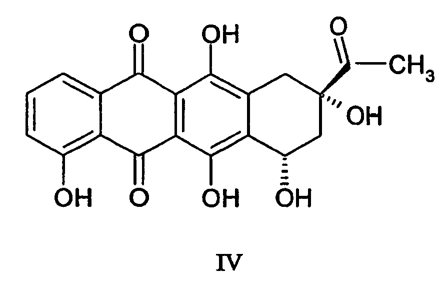 7-deoxy derivative of formula V 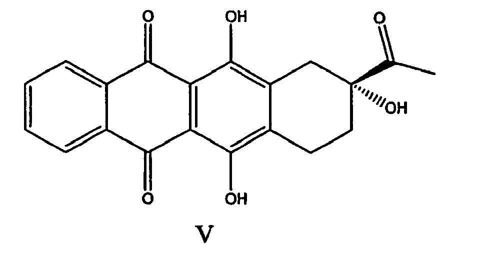 and idarubicin aglycone bis-anhydro of formula VI.

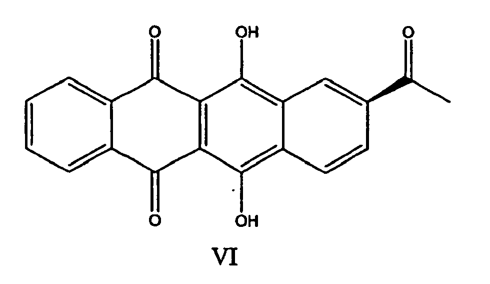

Preferably, idarubicin aglycone prepared by the above process contains less than 0.1 % of 4-hydroxy derivative of formula IV.

The present invention also provides idarubicin aglycone of formula I containing less than 0.1 % area by HPLC of 4-hydroxy derivative of formula IV.

Idarubicin aglycone obtained by the process of this invention may be converted to pharmaceutically acceptable salts of idarubicin preferably, idarubicin hydrochloride, for example, according to the process described in US patent No. 4,077,988.

The processes of producing idarubicin aglycone described herein may be altered in size according to the amount of product desired or the nature of the equipment used. The amounts of reagents indicated in the Examples are based on a typical production batch, but such amounts can be varied, depending on the amount of product desired or the nature of the equipment used. Reaction temperatures, times, and quantities of chemicals indicated may be varied somewhat to increase process efficiency without adversely affecting product characteristics.

The present invention may be better understood by reference to the following Examples.

### EXAMPLES

### Example 1 :

A solution of 4-demethyldaunomycinone-4-triflate¹ (50.00 g, 96.7 mmol) and dichlorobis(triphenylphosphine)pafladiwn(II) (1.36 g, 1.93 mmol) in dimethylformamide (1130 ml) was prepared at 24°C, under a nitrogen stream. A solution of triethylsilane (11.89 g, 102.2 mmol), 2,6-dimethylpyridine (11.93 g, 111.0 mmol) and water (4.35 g, 242.0 mmol) in dimethylformamide (1066 ml) was added dropwise in 1 h. The reaction was monitored by HPLC. When the conversion of 4-demethyldaunomycinone-4-triflate arrested (in about 8 h., with 90-98% of conversion), a solution of triethylsilane (0.24 g, 2.1 mmol) in dimethylformamide (14 ml) was added. After 2 h., the reaction mixture was treated with 37% hydrochloric acid (9.3 ml) and H₂O (4400 ml) was then added dropwise over 1.5 h. The crude product was recovered by filtration, washed with a mixture of H₂O (440 ml) and methanol (440 ml), and dried at 50°C under vacuum. Crude 4-demethoxydaunomycinone (33.8 g, recovery 94.9%, HPLC purity 95.5%, containing 7-deoxy-4-demethoxydaunomycinone 0.4 %, Idarubicin aglycone bis-anhydro 0.14 % and 4-demethyl-daunomicinone 0.28%, which is the 4-hydroxy derivative, was suspended in methylene chloride (1010 ml) and acetone (440 ml) and the mixture was refluxed for 3 hours. Diisopropylether (930 ml) was then added dropwise, keeping the mixture under reflux. The mixture was concentrated under vacuum to a residue volume of 2.01, diisopropylether (1930 ml) was added dropwise and the resulting mixture was kept under reflux for 0.5 h. The mixture was then cooled to room temperature, kept at this temperature for 3 hours, and filtered to obtain 30.10 g of crystal 4-demethoxydaunomycinone. Overall yield 84.5 %, HPLC purity 99% (containing 7-deoxy-4-demethoxydaunomycinone 0.14 %, and 4-demethyl-daunomicinone 0.23%, which is the 4-hydroxy derivative). ¹ Starting material contains 0.17% of 4-demethyl-daunomicinone, which is the 4-hydroxy derivative.

### Example 2:

A solution of 4-demethyldaunomycinone-4-triflate¹ (50.00 g, 96.7 mmol) and dichlorobis(triphenylphosphine)palladium(II) (1.36 g, 1.93 mmol) in dimethylformamide (1130 ml) was prepared at 21°C, under a nitrogen stream. A solution of triethylsilane (11.24 g, 96.7 mmol), 2,6-dimethylpyridine (11.93 g, 111.0 mmol) and water (1.74 g, 96.7 mmol) in dimethylformamide (1066 ml) was added dropwise over 20 minutes. When the conversion of 4-demethyldaunomycinone-4-triflate arrested (95% conversion), a solution of triethylsilane (0.56 g, 4.84 mmol) in dimethylformamide (14 ml) was added. After 2 h., the work up of the mixture and purification of crude product (the crude containing: 7-deoxy-4-demethoxydaunomycinone 0.28 % and 4-demethyl-daunomicinone 0.31 %) were accomplished as in Example 1, obtaining 31.08 g of crystal 4-demethoxydaunomycinone. Overall yield 88 %, HPLC purity 98.5% (containing 7-deoxy-4-demethoxydaunomycinone 0.12 % and 4-demethyl-daunomicinone 0.3%)
¹Starting material contains 0.25 % of 4-demethyl-daunomicinone.

### Example 3:

A solution of 4-demethyldaunomycinone4-triflate¹ (50.00 g, 96.7 mmol) and dichlorobis(triphenylphosphine)palladium(II) (1.36 g, 1.93 mmol) in dimethylformamide (1130 ml) was prepared at 24°C, under a nitrogen stream. A solution of triethylsilane (10.68 g, 91.85 mmol), 2,6-dimethylpyridine (11.93 g, 111.0 mmol) and water (0.87 g, 48.4 mmol) in dimethylformamide (1066 ml) was added (all at once). When the conversion of 4-demethyldaunomycinone-4-triflate arrested (95% conversion), a solution of triethylsilane (0.56 g, 4.82 mmol) in dimethylformamide (14 ml) was added. After 2 h., the work up of the mixture and purification of crude product (purity 90.9 % by HPLC, containing 7-deoxy-4-demethoxydaunomycinone 0.69 %, Idarubicin aglycone bis-anhydro 0.2 %, 4-demethyldaunomycinone-4-triflate 6.6% and 4-demethyl-daunomicinone 0.39%) were accomplished as in Example 1, obtaining 26.6 g of crystal 4-demethoxydaunomycinone. Overall yield 74.7 %, HPLC purity 97.9 %.(containing 7-deoxy-4-demethoxydaunomycinone 0.24 % and 4-demethyl-daunomicinone 0.35 %).
¹ Starting material contains 0.25 % of 4-demethyl-daunomicinone

### Example 4:

A solution of 4-demethyldaunomycinone-4-triflate¹ (50.00 g, 96.7 mmol) and dichlorobis(triphenylphosphine)palladium(D) (1.36 g,1.93 mmol) in dimethylformamide (1130 ml) was prepared at 15°C, under a nitrogen stream. A solution of triethylsilane (12.93 g, 111.2 mmol) and 2,6-dimethylpyridine (11.93 g, 111.0 mmol) in dimethylformamide (1080 ml) was added dropwise over 1.5 h. After 3.5 hours, the reaction was complete, and the work up of the mixture and purification of crude product (purity 94%, containing 7-deoxy-4-demethoxydaunomycinone 3.0%, Idarubicin aglycone bis-anhydro 0.1% and 4-demethyl-daunomicinone 0.44%) were accomplished as in Example 1, affording 4-demethoxydaunomycinone in 88.2 % overall yield and in purity of 98 %by HPLC. (containing 7-deoxy-4-demethoxydaunomycinone 1.0 % and 4-demethyl-daunomicinone 0.28%)
¹ Starting material contains 0.25% of 4-demethyl-daunomicinone

### Example 5:

A solution of 4-demethyldaunomycinone-4-triflate¹ (1.88 g, 3.64 mmol) and dichlorobis(triphenylphosphine)palladium(II) (0.0513 g, 0.0731 mmol) in dimethylformamide (44 ml) was prepared at 46°C, under a nitrogen stream. A solution of triethylsilane (0.4645 g, 4.00 mmol) and triethylamine (0.4074 g, 4.02 mmol) in dimethylformamide (34 ml) was added dropwise over 1 h. When the conversion of 4-demethyldaunomycinone-4-triflate arrested, a solution of triethylsilane (0.0524 g, 0.45 mmol) and triethylamine (0.0423 g, 0.42 mmol) in dimethylformamide (10 ml) was added. After 0.5 h, the reaction mixture was cooled to 28°C, treated with glacial acetic acid (1.61 ml), heated to 50°C, and treated with hydrochloric acid 37% (0.75 ml). After 1.5 h, H₂O (140 ml) was added dropwise (1.5 h) at 50°C and the mixture was slowly cooled to room temperature. The crude product was recovered by filtration, washed with a mixture of H₂O (14 ml) and methanol (14 ml) and dried at 50°C under vacuum, affording crude 1.05 g (73% yield)of 4-demethoxydaunomycinone HPLC purity 93.3%, containing 7-deoxy-4-demethoxydaunomycinone less than 0.1%, 4-demethyldaunomycinone 0.2%, Idarubicin aglycone bis-anhydro 3.6%).
¹ Starting material contains less than 0.10 % of 4-demethyl-daunomicinone.

### Example 6:

A solution of 4-demethyldaunomycinone-4-triflate¹ (50.00 g, 96.7 mmol) and dichlorobis(triphenylphosphine)palladium(II) (1.36 g, 1.93 mmol) in dimethylformamide (1100 ml) was prepared at 46°C, under a nitrogen stream. A solution of triethylsilane (13.50 g, 116.1 mmol) and 2,6-dimethylpyridine (11.41 g, 106.5 mmol) in dimethylformamide (1025 ml) was added dropwise over 1 h. When the conversion of 4-demethyldaunomycinone-4-triflate arrested (40 min.), a solution of triethylsilane (0.57 g, 4.92 mmol) in dimethylformamide (50 ml) was added. After 20 minutes, the reaction was complete and the work up of the mixture was accomplished as in Example 5, affording crude 4-demethoxydaunomycinone (32.6 g, HPLC purity 95.0 %, containing 7-deoxy-4-demethoxydaunomycinone 0.47% Idarubicin aglycone bis-anhydro 0.3%, 4-demethyldaunomycinone-4-triflate 0.4% and 4-demethyl-daunomicinone less than 0.1%, 87% yield).

The product was then crystallized using a 4:6 mixture of toluene and THF (11) to dissolve the product at reflux followed by cooling for 2 hours to 25°C and maintaining the mixture overnight at this temperature. After filtration, washing and drying, the pure product was obtained. HPLC purity 98.7% (containing 7-deoxy-4-demethoxydaunomycinone 0.11 %, and 4-demethyl-daunomicinone less than 0.10%).
¹ Starting material contains less than 0.10 % of 4-demethyl-daunomicinone.

### Example 7:

To a solution of 4-demethoxy-daunomycinone (1 g) in 230 ml of anhydrous chloroform, (2.2 g) of 2,3,6-trideoxy-3-trifluoroacetamido-4-O-trifluoroacetyl-oc-L-lyxopyranosyl-chloride, (2 g) of HgO, (0.5 g) of HgBr₂, and (15 g) of 5A molecular sieve were added under stirring. The suspension was stirred in the dark for 24 hours, filtered, concentrated in vacuo and the residue dissolved in 350 ml of methanol. The solution was left overnight at room temperature. After evaporation of the solvent the residue was chromatographed on (20 g) silica gel, eluting first with chloroform: acetone 19:1 to give 0.55 g of a (-)-daunosaminyl-4-demethoxydaunomycinone-N-trifluoroacetate, which was then dissolved in 40 ml of 0.1N NaOH and kept at room temperature for 30 minutes. The solution was brought to pH 8 with HCl, and extracted with chloroform. Evaporation of the left a residue that was taken up in a little chloroform-methanol. Methanolic 0.1N HCl was added to adjust the pH to 4.5, after which sufficient ethylether was added to precipitate α-(-)-daunosaminyl-4-demethoxydaunomycinone hydrochloride.

### HPLC

Column: Hypersil BDS-C18 3µm - 100 x 4.6 mm
Mobil phase: Buffer (*): THF = 70:30
Flow: 1.5 ml/min
Temperature: 50°C
Wavelength: 254 nm
Run: 30 minutes
(*) Buffer: 2.9 g sodium laurylsulfate -1000 ml of water - 2.3 g phosphoric acid 85%

| Product | Retention time | RRT |
|---|---|---|
| 4-demethyl-daunomycinone-4-Triflate | 9.10 | 2.4 |
| 4-demethoxy-daunomycinone | 3.75 | 1.0 |
| 4-demethyl-daunomycinone | 4.44 | 1.2 |
| 7-deoxy-4-demethoxy-daunomycinone | 6.09 | 1.6 |
| Idarubicin aglycone bis-anhydro | 13.77 | 3.7 |

## Claims

1. A process for the preparation of idarubicin aglycone (4-demethoxydaunomycinone) of formula I, 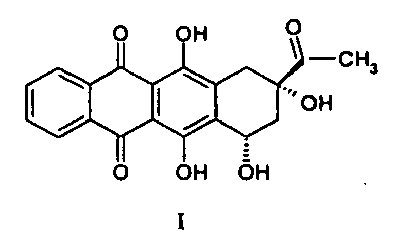 comprising the steps of
(a) combining the corresponding sulfonate of formula II, 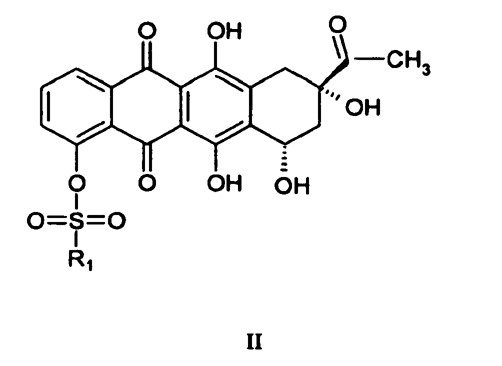 with a first polar aprotic organic solvent and with a metal catalyst or co-catalyst to obtain a first solution;
(b) combining 0.9 to 1.3 mole equivalents of the silyl reagent of the formula R₂R₃R₄SiH with a base, and a second polar aprotic organic solvent, with or without a protic solvent to obtain a second solution;
(c) adding the second solution of step (b) to the first solution of step (a) to obtain a mixture, and
(d) adding a solution of the silyl reagent of the formula R₂R₃R₄SiH in a second polar aprotic organic solvent to the mixture of step (c), when no more than 96% area by HPLC of the sulfonate of formula II has reacted, and
(e) maintaining the mixture obtained in step (d) for at least 20 minutes;
(f) quenching the mixture maintained in step (e), and
(g) recovering the idarubicin aglycone of the formula I; wherein
R₁ is C₁₋₁₀ alkyl, C₁₋₁₀ alkyl substituted with halogen, an aryl group, an aryl group substituted with halogen or an electron withdrawing group, and R₂, R₃, and R₄ are independently branched or linear C₁₋₄ alkyl, aryl, heteroaryl groups or polymethylsiloxane.

2. The process of claim 1, wherein R₁ is C₁₋₁₀ alkyl fully substituted with halogen.

3. The process of claim 2, wherein R₁ is CF₃.

4. The process of claim 1, wherein R₂, R₃ and R₄ are the same alkyl groups.

5. The process of claim 4, wherein R₂, R₃ and R₄ are ethyl groups.

6. The process of claim 1, wherein the amount of silyl reagent of the formula R₂R₃R₄SiH in step (b) is 1.15 mole equivalents per mole equivalent of the sulfonate of formula II.

7. The process of claim 1, wherein the second solution of step (b) is added to the first solution of step (a) in a dropwise manner.

8. The process of claim 7, wherein the dropwise addition is done over a period of 20 minutes to 1.5 hours.

9. The process of claim 1, wherein the second solution of step (b) also contains a protic solvent.

10. The process of claim 9, wherein the protic solvent is either C₁₋₅ alcohol or water.

11. The process of claim 10, wherein the C₁₋₅ alcohol is methanol.

12. The process of claim 9, wherein the protic solvent is water.

13. The process of claim 9, wherein the amount of the protic solvent is 0.1 mole equivalents to 5 mole equivalents per mole equivalent of the sulfonate of formula II.

14. The process of claim 1, wherein the temperature in step (a) is from 10°C to 46°C.

15. The process of claim 1, wherein the first polar aprotic organic solvent used in step (a) is selected from the group consisting of amide, ether and ketone.

16. The process of claim 15, wherein the first polar aprotic organic solvent is selected from the group consisting of dimethylformamide (DMF), dimethylacetamide, N-methylpyrrolidinone (NMP), tetrahydrofuran (THF), 2-methyl-THF, N-methylpiperidone and acetone.

17. The process of claim 16, wherein the first polar aprotic organic solvent is DMF.

18. The process of claim 1, wherein a metal co-catalyst is used in step (a).

19. The process of claim 18, wherein the metal co-catalyst is dichlorobis(triphenylphosphine)Ni(II), dichlorobis(triphenylphosphine)Pd(II), or Pd(II)(OAc)₂ in the presence of triphenyl phosphine.

20. The process of claim 19, wherein the metal co-catalyst is dichlorobis(triphenylphosphine)Pd(II).

21. The process of claim 1, wherein the base in step (b) is a weak organic base.

22. The process of claim 21, wherein the base is a hindered weak organic base.

23. The process of claim 22, wherein the base is selected from the group consisting of pyridine, 2,6-dimethylpyridine, diisopropylethylamine, triethylamine, tributylamine, imidazole.

24. The process of claim 23, wherein the base is 2,6-dimethylpyridine.

25. The process of claim 1, wherein the second polar aprotic organic solvent in step (b) is the same as the first aprotic organic solvent in step (a).

26. The process of claim 1, wherein the mixture obtained in step (d) is maintained for 20 minutes to 2 hours, depending on the reaction temperature.

27. The process of claim 1, wherein the quenching reagent used in step (f) is an acidic aqueous solution.

28. The process of claim 27 , wherein the acidic aqueous solution is selected from the group consisting of HCl, acetic acid, and ammonium chloride.

29. The process of claim 28, wherein the acidic aqueous solution is aqueous HCl.

30. The process of claim 1, further comprising crystallization of the product of step (g) from a mixture of a solvent and an anti-solvent.

31. The process of claim 30, wherein the solvent is a polar organic solvent.

32. The process of claim 31, wherein the polar organic solvent is selected from the group consisting of dichloromethane, acetone, acetonitrile and THF.

33. The process of claim 30, wherein the anti-solvent is a non-polar organic solvent.

34. The process of claim 33, wherein the non-polar organic solvent is either diisopropylether or toluene.

35. The process of claim 30, wherein the mixture of a solvent and an anti solvent contains acetonitrile with diisopropylether.

36. The process of claim 30, wherein the mixture of a solvent and an anti solvent contains THF with toluene.

37. The process of claim 30, wherein idarubicin aglycone contains less than 1% area by HPLC, of one of: the 4-hydroxy derivative of formula IV, 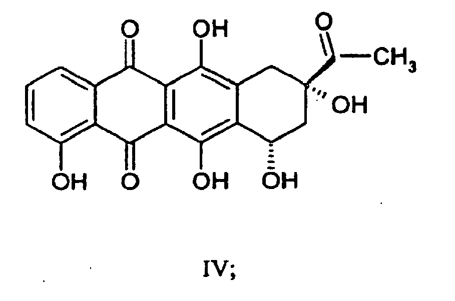 the 7-deoxy derivative of formula V, 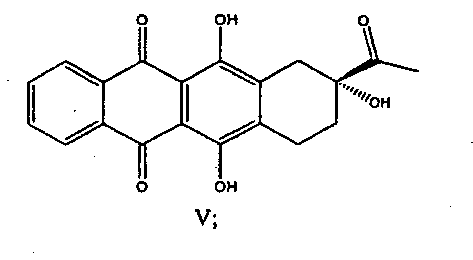 and the idarubicin aglycone bis-anhydro of formula VI,

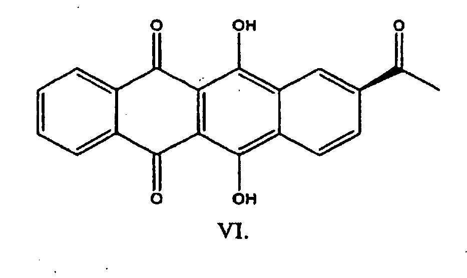

38. The process of claim 37, wherein idarubicin aglycone contains less than 0.1% area by HPLC, of the 4-hydroxy derivative of formula IV,

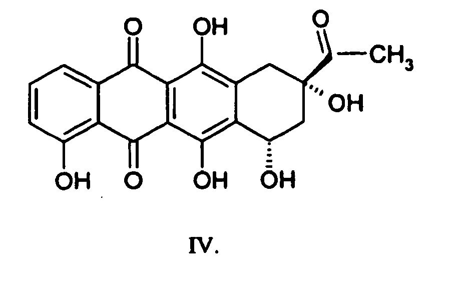

39. The process of any one of claims 1 to 38, further comprising converting the idarubicin aglycone to idarubicin hydrochloride.

## Patentansprüche

1. Verfahren zur Herstellung von Idarubicinaglykon (4-Desmethoxydaunomycinon) der Formel I 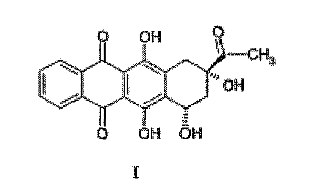 mit den Stufen, bei denen man
(a) das korrespondierende Sulfonat der Formel II 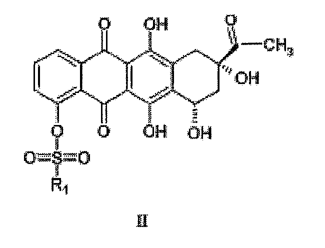 mit einem ersten polaren aprotischen organischen Lösungsmittel und mit einem Metallkatalysator oder einem Co-Katalysator kombiniert, um eine erste Lösung zu erhalten,
(b) 0,9 bis 1,3 Moläquivalente des Silylreagens mit der Formel R₂R₃R₄SiH mit einer Base und mit einem zweiten polaren aprotischen organischen Lösungsmittel mit oder ohne einem protischen Lösungsmittel kombiniert, um eine zweite Lösung zu erhalten,
(c) die zweite Lösung von Stufe (b) zu der ersten Lösung von Stufe (a) zugibt, um ein Gemisch zu erhalten, und
(d) eine Lösung des Silylreagens mit der Formel R₂R₃R₄SiH in einem zweiten polaren aprotischen Lösungsmittel zu dem Gemisch von Stufe (c) zugibt, wenn nicht mehr als 96 % Fläche anhand von HPLC des Sulfonats der Formel II reagiert hat, und
(e) das in Stufe (d) erhaltene Gemisch für wenigstens 20 Minuten aufrecht erhält,
(f) das in Stufe (e) erhaltene Gemisch quencht und
(g) das Idarubicinaglykon der Formel I gewinnt, wobei R₁ eine C₁₋₁₀-Alkyl-, eine mit Halogen substituierte C₁₋₁₀-Alkyl-, eine Aryl-Gruppe, eine mit Halogen substituierte Aryl-Gruppe oder eine Elektronen ziehende Gruppe ist, und wobei R₂, R₃ und R₄ unabhängig voneinander verzweigte oder gerade C₁₋₄-Alkyl-, Aryl-, Heteroaryl-Gruppen oder Polymethylsiloxan sind.

2. Verfahren nach Anspruch 1, wobei R₁ ein vollständig mit Halogen substituiertes C₁₋₁₀-Alkyl ist.

3. Verfahren nach Anspruch 2, wobei R₁ CF₃ ist.

4. Verfahren nach Anspruch 1, wobei R₂, R₃ und R₄ die gleichen Alkylgruppen sind.

5. Verfahren nach Anspruch 4, wobei R₂, R₃ und R₄ Ethylgruppen sind.

6. Verfahren nach Anspruch 1, wobei die Menge an Silylreagens mit der Formel R₂R₃R₄SiH in Stufe (b) 1,15 Moläquivalente pro Moläquivalent des Sulfonats der Formel II ist.

7. Verfahren nach Anspruch 1, wobei die zweite Lösung von Stufe (b) zu der ersten Lösung von Stufe (a) tropfenweise zugegeben wird.

8. Verfahren nach Anspruch 7, wobei die tropfenweise Zugabe durchgeführt wird über einen Zeitraum von 20 Minuten bis 1,5 Stunden.

9. Verfahren nach Anspruch 1, wobei die zweite Lösung von Stufe (b) auch ein protisches Lösungsmittel enthält.

10. Verfahren nach Anspruch 9, wobei das protische Lösungsmittel entweder C₁₋₅-Alkohol oder Wasser ist.

11. Verfahren nach Anspruch 10, wobei der C₁₋₅-Alkohol Methanol ist.

12. Verfahren nach Anspruch 9, wobei das protische Lösungsmittel Wasser ist.

13. Verfahren nach Anspruch 9, wobei die Menge des protischen Lösungsmittels 0,1 Moläquivalente bis 5 Moläquivalente pro Moläquivalent des Sulfonats von Formel II beträgt.

14. Verfahren nach Anspruch 1, wobei die Temperatur in Stufe (a) von 10 °C bis 46 °C beträgt.

15. Verfahren nach Anspruch 1, wobei das erste polare aprotische organische Lösungsmittel, welches in Stufe (a) verwendet wird, ausgewählt ist aus der Gruppe, bestehend aus Amid, Ether und Keton.

16. Verfahren nach Anspruch 15, wobei das erste polare aprotische organische Lösungsmittel ausgewählt ist aus der Gruppe, bestehend aus Dimethylformamid (DMF), Dimethylacetamid, N-Methylpyrrolidinon (NMP), Tetrahydrofuran (THF), 2-Methyl-THF, N-Methylpiperidon und Aceton.

17. Verfahren nach Anspruch 16, wobei das erste polare aprotische organische Lösungsmittel DMF ist.

18. Verfahren nach Anspruch 1, wobei ein Metall-Co-Katalysator in Stufe (a) verwendet wird.

19. Verfahren nach Anspruch 18, wobei der Metall-Co-Katalysator Dichlor-bis(triphenylphosphin)Ni(II), Dichlorbis(triphenylphosphin)Pd(II) oder Pd(II)(OAc)₂ in der Gegenwart von Triphenylphosphin ist.

20. Verfahren nach Anspruch 19, wobei der Metall-Co-Katalysator Dichlor-bis(triphenylphosphin)Pd(II) ist.

21. Verfahren nach Anspruch 1, wobei die Base in Stufe (b) eine schwache organische Base ist.

22. Verfahren nach Anspruch 21, wobei die Base eine gehinderte schwache organische Base ist.

23. Verfahren nach Anspruch 22, wobei die Base ausgewählt ist aus der Gruppe bestehend aus Pyridin, 2,6-Dimethylpyridin, Diisopropylethylamin, Triethylamin, Tributylamin, Imidazol.

24. Verfahren nach Anspruch 23, wobei die Base 2,6-Dimethylpyridin ist.

25. Verfahren nach Anspruch 1, wobei das zweite polare aprotische organische Lösungsmittel in Stufe (b) das gleiche ist wie das erste aprotische organische Lösungsmittel in Stufe (a).

26. Verfahren nach Anspruch 1, wobei das Gemisch, welches in Stufe (d) erhalten wird, in Abhängigkeit von der Reaktionstemperatur für 20 Minuten bis 2 Stunden aufrecht erhalten wird.

27. Verfahren nach Anspruch 1, wobei das Quench-Reagens, welches in Stufe (f) verwendet wird, eine saure wässrige Lösung ist.

28. Verfahren nach Anspruch 27, wobei die saure wässrige Lösung ausgewählt ist aus der Gruppe, bestehend aus HCl, Essigsäure und Ammoniumchlorid.

29. Verfahren nach Anspruch 28, wobei die saure wässrige Lösung wässrige HCl ist.

30. Verfahren nach Anspruch 1, bei dem man weiterhin das Produkt von Stufe (g) aus einem Gemisch eines Lösungsmittels und eines Anti-Lösungsmittels kristallisiert.

31. Verfahren nach Anspruch 30, wobei das Lösungsmittel ein polares organisches Lösungsmittel ist.

32. Verfahren nach Anspruch 31, wobei das polare organische Lösungsmittel ausgewählt ist aus der Gruppe, bestehend aus Dichlormethan, Aceton, Acetonitril und THF.

33. Verfahren nach Anspruch 30, wobei das Anti-Lösungsmittel ein nicht-polares organisches Lösungsmittel ist.

34. Verfahren nach Anspruch 33, wobei das nicht-polare organische Lösungsmittel entweder Diisopropylether oder Toluen ist.

35. Verfahren nach Anspruch 30, wobei das Gemisch eines Lösungsmittels und eines Anti-Lösungsmittels Acetonitril mit Diisopropylether enthält.

36. Verfahren nach Anspruch 30, wobei das Gemisch eines Lösungsmittels und eines Anti-Lösungsmittels THF mit Toluen enthält.

37. Verfahren nach Anspruch 30, wobei das Idarubicinaglykon weniger als 1 % Fläche anhand von HPLC von einem von: das 4-Hydroxy-Derivat von Formel IV 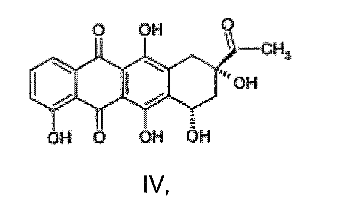 das 7-Desoxyderivat von Formel V 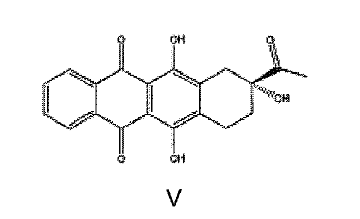 und das Bis-Anhydro-Idarubicinaglykon von Formel VI 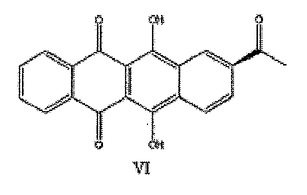 enthält.

38. Verfahren nach Anspruch 37, wobei das Idarubicinaglykon weniger als etwa 0,1 % Fläche anhand von HPLC des 4-Hydroxy-Derivats von Formel IV 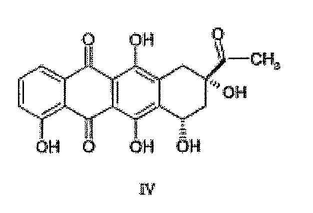 enthält.

39. Verfahren nach einem der Ansprüche 1 bis 38, bei dem man weiterhin das Idarubicinaglykon in Idarubicinhydrochlorid umwandelt.

## Revendications

1. Procédé pour la préparation d'aglycone d'idarubicine (4-déméthoxydaunomycinone) de formule 1 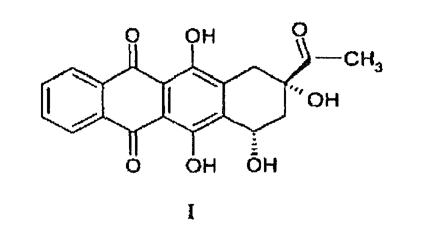 comprenant les étapes de
(a) combiner le sulfonate de formule II correspondant, 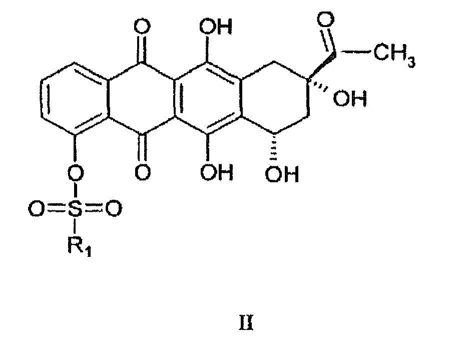 avec un premier solvant organique aprotique polaire et avec un catalyseur ou cocatalyseur métallique pour obtenir une première solution ;
(b) combiner 0,9 à 1,3 équivalent molaire de réactif silyle de formule R₂R₃R₄SiH avec une base, et un second solvant organique aprotique polaire, avec ou sans solvant protique pour obtenir une seconde solution ;
(c) ajouter la seconde solution de l'étape (b) à la première solution de l'étape (a) pour obtenir un mélange, et
(d) ajouter une solution du réactif silyle de formule R₂R₃R₄SiH dans un second solvant organique aprotique polaire au mélange de l'étape (c), quand pas plus de 96 % d'aire par CLHP du sulfonate de formule II ont réagi, et
(e) maintenir le mélange obtenu dans l'étape (d) pendant au moins 20 minutes ;
(f) fixer le mélange maintenu dans l'étape (e), et
(g) récupérer l'aglycone d'idarubicine de formule I ; où
R₁ est C₁₋₁₀ alkyl, C₁₋₁₀ alkyle substitué avec halogène, un groupe aryle, un groupe aryle substitué avec halogène ou un groupe attracteur d'électrons, et R₂, R₃ et R₄ sont indépendamment des groupes C₁₋₄ alkyle linéaires ou ramifiés, aryle, hétéroaryle ou un polyméthylsiloxane.

2. Procédé selon la revendication 1 où R₁ est C₁₋₁₀ alkyle totalement substitué avec halogène.

3. Procédé selon la revendication 2 où R₁ est CF₃.

4. Procédé selon la revendication 1 où R₂, R₃ et R₄ sont les mêmes groupes alkyle.

5. Procédé selon la revendication 4 où R₂, R₃ et R₄ sont des groupes éthyle.

6. Procédé selon la revendication 1 où la quantité de réactif silyle de formule R₂R₃R₄SiH dans l'étape (b) est 1,15 équivalent molaire par équivalent molaire de sulfonate de formule II.

7. Procédé selon la revendication 1 où la seconde solution de l'étape (b) est ajoutée à la première solution de l'étape (a) goutte à goutte.

8. Procédé selon la revendication 7 où l'addition goutte à goutte est accomplie pendant une durée de 20 minutes à 1,5 heure.

9. Procédé selon la revendication 1 où la seconde solution de l'étape (b) contient aussi un solvant protique.

10. Procédé selon la revendication 9 où le solvant protique est un C₁₋₅ alcool ou l'eau.

11. Procédé selon la revendication 10 où le C₁₋₅ alcool est le méthanol.

12. Procédé selon la revendication 9 où le solvant protique est l'eau.

13. Procédé selon la revendication 9 où la quantité de solvant protique est 0,1 équivalent molaire à 5 équivalents molaires par équivalent molaire de sulfonate de formule II.

14. Procédé selon la revendication 1 où la température dans l'étape (a) est de 10°C à 46°C.

15. Procédé selon la revendication 1 où le premier solvant organique aprotique polaire utilisé dans l'étape (a) est choisi dans le groupe consistant en un amide, un éther et une cétone.

16. Procédé selon la revendication 15 où le premier solvant organique aprotique polaire est choisi dans le groupe consistant en le diméthylformamide (DMF), le diméthylacétamide, la N-méthylpyrrolidinone (NMP), le tétrahydrofurane (THF), le 2-méthyl-THF, la N-méthylpipéridone et l'acétone.

17. Procédé selon la revendication 16 où le premier solvant organique aprotique polaire est le DMF.

18. Procédé selon la revendication 1 où un cocatalyseur métallique est utilisé dans l'étape (a).

19. Procédé selon la revendication 18 où le cocatalyseur métallique est le dichlorobis(triphénylphosphine)Ni(II), le dichlorobis(triphénylphosphine)Pd(II) ou Pd(II)(OAc)₂ en présence de triphénylphosphine.

20. Procédé selon la revendication 19 où le cocatalyseur métallique est le dichlorobis(triphénylphosphine)Pd(II).

21. Procédé selon la revendication 1 où la base dans l'étape (b) est une base organique faible.

22. Procédé selon la revendication 21 où la base est une base organique faible à empêchement stérique.

23. Procédé selon la revendication 22 où la base est choisie dans le groupe consistant en la pyridine, la 2,6-diméthylpyridine, la diisopropyléthylamine, la triéthylamine, la tributylamine, l'imidazole.

24. Procédé selon la revendication 23 où la base est la 2,6-diméthylpyridine.

25. Procédé selon la revendication 1 où le second solvant organique aprotique polaire dans l'étape (b) est le même que le premier solvant organique aprotique dans l'étape (a).

26. Procédé selon la revendication 1 où le mélange obtenu dans l'étape (d) est maintenu pendant 20 minutes à 2 heures, selon la température de réaction.

27. Procédé selon la revendication 1 où le réactif de fixation utilisé dans l'étape (f) est une solution aqueuse acide.

28. Procédé selon la revendication 27 où la solution aqueuse acide est choisie dans le groupe consistant en HCl, l'acide acétique et le chlorure d'ammonium.

29. Procédé selon la revendication 28 où la solution aqueuse acide est HCl aqueux.

30. Procédé selon la revendication 1 comprenant en outre la cristallisation du produit de l'étape (g) dans un mélange d'un solvant et d'un antisolvant.

31. Procédé selon la revendication 30 où le solvant est un solvant organique polaire.

32. Procédé selon la revendication 31 où le solvant organique polaire est choisi dans le groupe consistant en le dichlorométhane, l'acétone, l'acétonitrile et le THF.

33. Procédé selon la revendication 30 où l'antisolvant est un solvant organique non polaire.

34. Procédé selon la revendication 33 où le solvant organique non polaire est le diisopropyléther ou le toluène.

35. Procédé selon la revendication 30 où le mélange d'un solvant et d'un antisolvant contient de l'acétonitrile avec du diisopropyléther.

36. Procédé selon la revendication 30 où le mélange d'un solvant et d'un antisolvant contient du THF avec du toluène.

37. Procédé selon la revendication 30 où l'aglycone d'idarubicine contient moins de 1 % d'aire par CLHP, de l'un de : le dérivé 4-hydroxy de formule IV 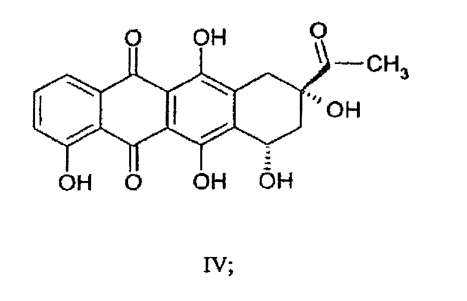 le dérivé 7-désoxy de formule V 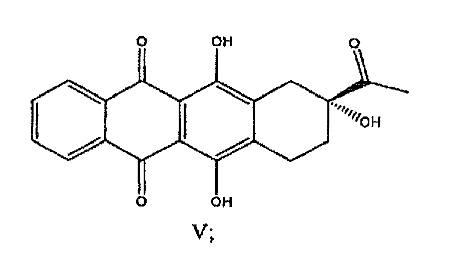 et l'aglycone bis-anhydro d'idarubicine de formule VI

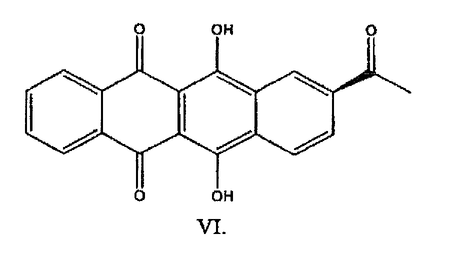

38. Procédé selon la revendication 37 où l'aglycone d'idarubicine contient moins de 0,1 % d'aire par CLHP, du dérivé 4-hydroxy de formule IV

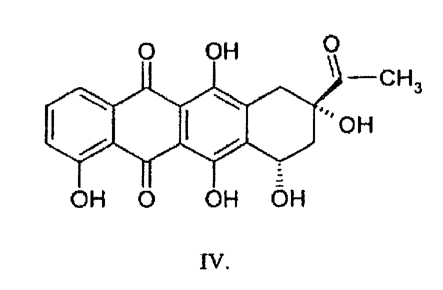

39. Procédé selon l'une quelconque des revendications 1 à 38 comprenant en outre la conversion de l'aglycone d'idarubicine en chlorhydrate d'idarubicine.
